(19) Europäisches Patentamt European Patent Office Office européen des brevets

(11) **EP 2 125 112 B1**

(12) **FASCICULE DE BREVET EUROPEEN**

(45) Date de publication et mention
de la délivrance du brevet:
**19.09.2012   Bulletin 2012/38**

(21) Numéro de dépôt: **07871959.8**

(22) Date de dépôt: **18.12.2007**

(51) Int Cl.:
***A61N 7/02*** *(2006.01)*

(86) Numéro de dépôt international:
**PCT/FR2007/052545**

(87) Numéro de publication internationale:
**WO 2008/081147 (10.07.2008 Gazette 2008/28)**

(54) **TETE DE TRAITEMENT THERAPEUTIQUE**

THERAPEUTISCHER BEHANDLUNGSKOPF

THERAPEUTIC TREATMENT HEAD

(84) Etats contractants désignés:
**AT BE BG CH CY CZ DE DK EE ES FI FR GB GR
HU IE IS IT LI LT LU LV MC MT NL PL PT RO SE
SI SK TR**

(30) Priorité:   **18.12.2006   FR 0655602**

(43) Date de publication de la demande:
**02.12.2009   Bulletin 2009/49**

(73) Titulaire: **Theraclion
75014 Paris (FR)**

(72) Inventeurs:
• **LACOSTE, François
F-75015 Paris (FR)**
• **PECHOUX, Thierry
F-75019 Paris (FR)**

(74) Mandataire: **CAPRI
33, rue de Naples
75008 Paris (FR)**

(56) Documents cités:
**EP-A1- 0 614 651      WO-A-02/05897
US-A- 5 759 162**

**Description**

La présente invention concerne une tête de traitement thérapeutique pour traiter des tissus vivants. . Par conséquent, le domaine d'application privilégié de la présente invention est celui des appareils, instruments, systèmes de traitement thérapeutique pour traiter les tissus vivants. Plus particulièrement, le domaine d'application plus restreint est celui du traitement thérapeutique ultrasonore.

On connaît déjà dans l'art antérieur différents types d'appareils de traitement thérapeutique ultrasonore utilisant des têtes de traitement thérapeutique mettant en oeuvre un transducteur thérapeutique ultrasonore émettant des ultrasons, par exemple du type HIFU (High Intensity Focused Ultrasound). En général, le transducteur thérapeutique ultrasonore est monté sur un boîtier de la tête qui forme une configuration concave ou en forme de dôme. Le transducteur thérapeutique ultrasonore est monté dans ce dôme concave et présente de ce fait également une forme concave en forme de dôme. En général, la tête de traitement comprend également une membrane souple déformable montée sur le boîtier de manière à former une chambre avec le boîtier et le transducteur. Cette chambre est conventionnellement remplie avec un liquide de couplage acoustique qui peut être de l'eau. Le liquide circule à travers la chambre entre une entrée et une sortie. Pour maintenir le liquide à une température constante relativement basse, il est souvent prévu des moyens de refroidissement entre la sortie et l'entrée de la chambre. Le transducteur est disposé sur le boîtier de manière à émettre des ultrasons, de préférence focalisés, à travers une zone de propagation de la chambre en direction de la membrane. En utilisation, la membrane est destinée à venir en contact d'une surface d'application des tissus à traiter, telle que la peau. Les ultrasons émis par le transducteur traversent donc la zone de propagation de la chambre, la membrane, la surface d'application du tissu et le tissu, et sont en général focalisés vers une cible située dans le tissu. Les ultrasons sont ainsi absorbés par le tissu, la puissance absorbée étant fonction de la capacité d'absorption du tissu et de l'intensité des ultrasons. Il en résulte une montée en température du tissu, qui dépend de la puissance absorbée, de la capacité calorifique du tissu et aussi des pertes thermiques, par exemple par conduction par le flux sanguin. Bien entendu, le liquide de couplage acoustique circulant à l'intérieur de la chambre permet d'évacuer une partie de la chaleur, ce qui évite de brûler les tissus. En outre, la membrane permet de faciliter le contact acoustique lorsqu'elle est en contact intime avec la surface d'application du tissu à traiter. Le document WO 02/05897 décrit une tête de traitement thérapeutique pour traiter des tissus vivants selon le préambule de la revendication 1.

Un des problèmes du traitement ultrasonore des tissus est le contrôle de l'absorption réelle du tissu, en particulier au niveau de l'interface entre la tête et le tissu, c'est-à-dire au niveau où la membrane touche la surface d'application du tissu à traiter. Lorsqu'il s'agit d'un traitement extracorporel, la surface d'application est la peau. Si une chaleur excessive est déposée au niveau de la peau, celle-ci va subir des dommages thermiques tels que des cloques ou des brûlures.

Un autre problème du traitement ultrasonore des tissus est le contrôle du contact acoustique entre la tête et le tissu à traiter. Il est en effet possible que la membrane ne soit pas en bon contact avec le tissu, ce qui se traduit par une réflexion de l'énergie ultrasonore sur la membrane, et de ce fait le traitement va être insuffisant. Dans certains cas, où la membrane n'est pas du tout en contact avec le tissu, il y a même risque de destruction du transducteur. Ceci peut notamment arriver lorsque la membrane est séparée de la peau par une couche d'air.

Un autre problème consiste au contrôle de l'énergie émise par le transducteur. En effet, une panne de la tête peut se produire et pour une raison quelconque le transducteur n'émet plus ou pas assez d'ultrasons. Le traitement thermique ne s'effectue alors pas de manière satisfaisante.

Un autre problème consiste à la détermination du séquencement optimum de l'application de l'énergie ultrasonore au tissu. En effet, avant application de la membrane sur la peau, celle-ci est à température corporelle. Il faut attendre quelques minutes pour que la peau et les tissus sous-cutanés soient suffisamment refroidis pour qu'un échauffement éventuel créé par l'onde acoustique ne se traduise pas par un dommage cutané ou sous-cutané.

En résumé, les buts de la présente invention sont d'éviter un endommagement thermique de la surface d'application (peau) du tissu à traiter, de détecter un mauvais contact de la tête avec la surface d'application du tissu à traiter et de détecter un mauvais fonctionnement du transducteur de la tête.

Pour ce faire, la présente invention propose une tête de traitement thérapeutique pour traiter des tissus vivants, la tête comprenant un boîtier, un transducteur thérapeutique ultrasonore monté sur le boîtier et une membrane montée sur le boîtier, la membrane étant destinée à venir en contact avec une surface d'application des tissus, telle que la peau, la membrane, le transducteur et le boîtier définissant ensemble une chambre remplie d'un liquide de couplage acoustique qui circule à travers la chambre entre une entrée et une sortie, le transducteur étant disposé de manière à émettre des ultrasons à travers une zone de propagation de cette chambre en direction de la membrane, caractérisée en ce qu'un premier capteur de température est disposé à proximité de l'entrée et un second capteur de température est disposé à proximité de la sortie, les capteurs délivrant des signaux représentatifs des températures. Ces signaux peuvent par exemple être utilisés pour établir un différentiel de températures du liquide de couplage dans la chambre, ce différentiel servant à déterminer indirectement la température de la surface d'application. En effet, la température de la surface d'application (peau) du tissu à traiter permet de savoir s'il y a un risque de brûlure, un mauvais contact de la tête et de la surface d'application ou un mauvais fonctionnement du transducteur. La première solution qui vient à l'esprit est de mesurer directement la température de la surface d'application en plaçant par exemple un capteur de température sur la membrane destinée à venir en contact de la surface d'application. Ceci serait peut être techniquement possible, mais les valeurs mesurées avec un tel capteur monté sur la membrane seraient erronées, du fait que le capteur serait monté dans la zone de propagation des ultrasons émis par le transducteur et qu'il absorberait une partie de l'énergie ultrasonore. Les valeurs de température mesurées seront donc erronées, puisque perturbées par l'interaction entre le capteur de température et le champ acoustique. Il n'est donc pas possible de mesurer directement la température de la surface d'application (peau) à l'aide d'un capteur de température monté sur la membrane. D'autre part, en plaçant un capteur de température hors de la zone de propagation des ultrasons, il n'est pas non plus possible de mesurer la température de la surface d'application, même indirectement. C'est pourquoi la présente invention prévoit deux capteurs de température, avantageusement situés de part et d'autre de la chambre. Avantageusement, les capteurs de température sont situés hors de la zone de propagation. Il est ainsi possible de mesurer de manière continue un différentiel de température et d'analyser les variations de ce différentiel de température en corrélation avec d'autres facteurs ou paramètres de manière à déterminer, déduire, calculer indirectement la température de la surface d'application (peau).

En général, la tête de traitement thérapeutique de l'invention est intégrée dans un appareil de traitement thérapeutique qui comprend avantageusement une électronique recevant les signaux de température délivrés par les capteurs de température pour établir un différentiel de température servant à déterminer la température de la surface d'application. Avantageusement, l'électronique calcule l'énergie calorifique absorbée ou rejetée par le liquide dans la chambre. Avantageusement, l'appareil de traitement comprend en outre un capteur de débit de liquide délivrant des signaux de débit à l'électronique pour calculer l'énergie calorique absorbée ou rejetée par le liquide dans la chambre. De préférence, l'appareil de traitement comprend en outre des moyens de refroidissement pour refroidir le liquide avant de l'injecter dans la chambre à travers l'entrée. Avantageusement, les moyens de refroidissement comprennent au moins une plaque de refroidissement exerçant une pression réglable sur une poche réservoir de liquide. L'électronique reçoit ainsi les valeurs des températures d'entrée et de sortie délivrées par les deux capteurs, et éventuellement les valeurs de débit de liquide reçues par le capteur de débit. En outre, l'électronique peut tenir compte de la température ambiante et des pertes thermiques du transducteur ultrasonore. Tous les transducteurs ultrasonores ont un rendement limité de l'ordre de 50 à 80%. Ceci signifie que 20 à 50% de l'énergie électrique apportée au transducteur est transformée en chaleur. Grâce à toutes ces valeurs mesurées et ces paramètres, l'électronique est capable de déterminer la température de la surface d'application du tissu à traiter.

Selon une autre caractéristique de l'invention, l'appareil peut comprendre au moins une pompe et un capteur de pression couplés à un régulateur de pression pour réguler la pression dans la chambre

Selon un aspect intéressant de l'invention, l'appareil de traitement comprend en outre des moyens de commutation du transducteur couplés à l'électronique, les moyens de commutation désactivant le transducteur lorsque le différentiel de température ou la température estimée de la surface d'application atteint une valeur plafond. Avantageusement, les moyens de commutation activent le transducteur lorsque le différentiel de température ou la température estimée de la surface d'application est redescendu à une valeur seuil prédéterminée indicative d'une température normale de la surface d'application.

Il est égalemet décrit un procédé de séquencement des phases d'activation d'un transducteur d'une tête de traitement telle que définie ci-dessus, dans lequel le transducteur est activé et désactivé en fonction de la température de la surface d'application indirectement déterminée à partir du différentiel de température mesuré. Le différentiel de température qui sert à déterminer indirectement la température de la surface d'application (peau) va ainsi servir de facteur déclenchant pour activer et désactiver le transducteur ultrasonore. Ainsi, le cycle de fonctionnement du transducteur est directement déterminé par le différentiel de température ou la température de la surface d'application indirectement déterminée à partir du différentiel de température. On évite ainsi tout risque d'endommagement de la surface d'application et tout risque de mauvais contact entre la membrane et la surface d'application.

Il est également décrit un procédé de détermination indirecte de la température de la surface d'application en contact d'une membrane d'une tête de traitement thérapeutique telle que définie ci-dessus, caractérisé en ce que le différentiel de température entre la sortie et l'entrée de la chambre est corrélé avec le débit de liquide à travers la chambre pour déterminer la température de la surface d'application. Avantageusement, le différentiel de température est corrélé avec la température ambiante pour déterminer la température de la surface d'application. Avantageusement, le différentiel de température est corrélé avec l'énergie du transducteur dissipée en chaleur dans le liquide de la chambre pour déterminer la température du débit de liquide de la surface d'application.

Les étapes de détermination indirecte de la température de la surface d'application à partir du différentiel de température, de la température ambiante et de la chaleur dissipée par le transducteur sont mises en oeuvre dans un logiciel approprié de l'électronique. Selon un mode opératoire avantageux, qui n'est pas un mode de l'invention, dans une phase initiale avant activation du transducteur, la membrane est mise en contact de la surface d'application jusqu'à ce que la température estimée de la surface d'application soit suffisamment stabilisée.

Grâce à la détermination indirecte de la température de la surface d'application (peau), il est ainsi possible de contrôler la température de la peau de manière à la refroidir suffisamment avant le début du traitement thérapeutique (activation du transducteur) pour éviter tout risque de brûlure.

Un principe intéressant de l'invention est de déterminer indirectement la température de la surface d'application des tissus vivants à traiter en mesurant un différentiel de température du liquide de couplage acoustique à l'intérieur de la chambre et en le corrélant avec d'autres paramètres extérieurs ou intérieurs qui agissent sur la température du liquide de couplage. Au final, le différentiel de température devient une valeur directement représentative de la température de la surface d'application.

L'invention sera maintenant plus amplement décrite en référence aux dessins joints donnant à titre d'exemple non limitatif un mode de réalisation de l'invention.

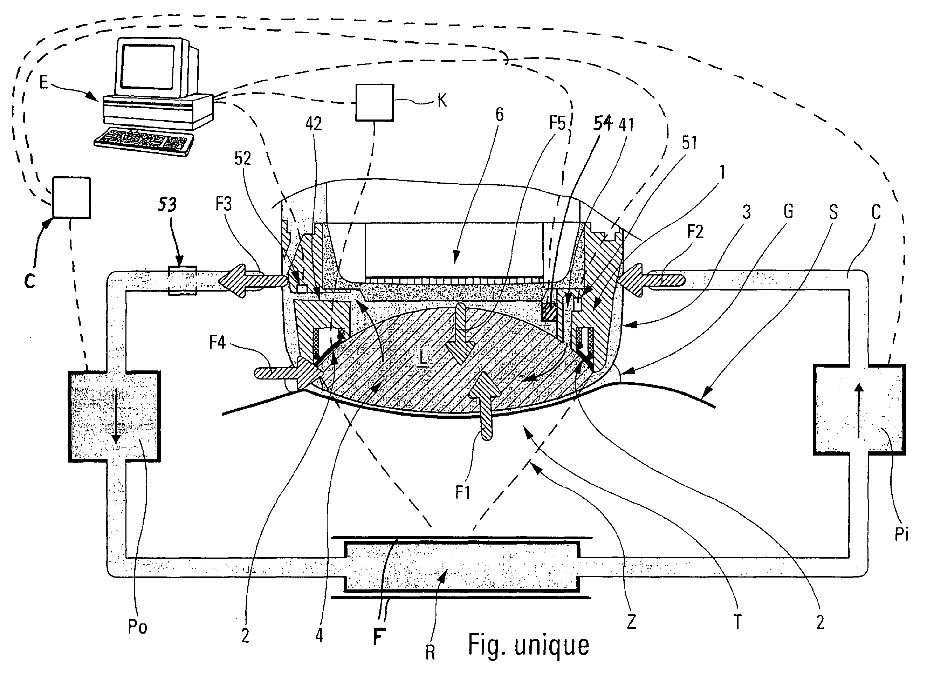

La figure unique est une représentation partielle schématique d'un appareil de traitement thérapeutique comprenant une tête de traitement thérapeutique selon l'invention associée à une électronique.

L'appareil de traitement thérapeutique de l'invention comprend une tête de traitement thérapeutique qui n'est représentée que partiellement sur la figure unique. En effet, il n'a été représenté que la partie inférieure de la tête qui est intéressante pour la présente invention. La tête comprend en outre une partie supérieure non représentée qui permet par exemple son montage sur une structure de support et sa connexion électrique ou VDI (voie-donnée-image). La partie basse de la tête est représentée sur la figure unique avec un circuit de liquide de couplage acoustique schématique. La tête, et éventuellement le circuit, est raccordée à une électronique de traitement E qui est ici symbolisée par un ordinateur. Les liaisons entre la tête et l'électronique sont représentées en traits pointillés. L'appareil comprend également un commutateur K qui est relié à la fois à la tête et à l'électronique E. La tête de traitement est ici représentée en contact avec une surface d'application S d'un tissu T à traiter. Dans la suite de la description, on considèrera que la surface d'application S est de la peau, bien qu'il puisse s'agir d'autres types de surfaces, comme des muqueuses ou un tissu sans peau, ni muqueuse.

La tête de traitement thérapeutique comprend un corps ou boîtier 1 qui peut être réalisé dans n'importe quel matériau approprié, comme du métal, ou avantageusement une matière comme du PEEK®. Il est avantageux d'utiliser pour le boîtier un matériau thermiquement isolant. Le boîtier 1 sert de support à de nombreux éléments constitutifs de la tête, comme par exemple un transducteur thérapeutique ultrasonore 2, une membrane souple déformable 3 et une sonde échographique 6. Le boîtier 1 définit également un canal d'entrée 41 et un canal de sortie 42. Le boîtier 1 présente une surface inférieure de configuration concave de forme sphérique ou de dôme. Le transducteur thérapeutique ultrasonore 2 est fixé sur cette configuration concave de manière à en occuper la majeure partie, bien que cela ne soit pas visible sur la figure unique. Ainsi, le transducteur 2 présente également une configuration en forme de dôme qui est interrompue au niveau d'une fenêtre dans laquelle est positionnée la sonde échographique 6. La sonde et la fenêtre sont toutefois optionnelles. En général, la sonde échographique 6 est une sonde allongée, par exemple à barrettes, qui est disposée dans une fenêtre allongée qui sépare le transducteur thérapeutique 2 en deux parties sensiblement symétriques. Le transducteur 2 s'étend avantageusement autour de la fenêtre dans laquelle est positionnée la sonde échographique 6. La sonde échographique 6 a bien entendu une fonction de visualisation échographique du tissu à traiter. Quant à la sonde thérapeutique 2, sa fonction est d'émettre des ultrasons, de préférence focalisés du type HIFU, vers une cible à l'intérieur du tissu T. On peut également remarquer sur la figure unique que les canaux 41 et 42 débouchent au niveau de la configuration concave du boîtier à proximité de la fenêtre de la sonde échographique 6.

La membrane souple déformable 3 est fixée à l'extérieur du boîtier 1 par tous moyens appropriés. La membrane s'étend devant la configuration concave du boîtier où sont montés le transducteur 2 et la sonde échographique 6. La membrane 3, le boîtier 1, le transducteur 2 et la sonde échographique 6 définissent ensemble une chambre 4 de volume variable, étant donné que la membrane 3 est déformable. Cette chambre 4 est remplie avec un liquide de couplage acoustique L qui peut être de l'eau ou un gel approprié. La chambre remplie de liquide avec sa membrane sont également désignées communément sous le terme de « ballonnet ». La chambre 4 communique avec l'extérieur à travers le canal d'entrée 41 et le canal de sortie 42. Les canaux 41 et 42 sont raccordés à un circuit C qui fait circuler le liquide de couplage acoustique L à travers la chambre 4. A la sortie du canal 42, le circuit peut par exemple être pourvu d'un capteur de débit 53 qui va mesurer le débit de liquide de couplage dans le circuit C. Le capteur de débit 53 délivre des signaux de débit qui sont envoyés à l'électronique E. Le circuit C comprend une poche réservoir R de liquide de couplage. Pour la réfrigérer, cette poche est insérée dans un bain d'eau froide ou entre deux plaques F refroidies, par exemple par des éléments thermoélectriques à effet Peltier. Les deux plaques F peuvent être articulées, ce qui permet d'ajuster la pression dans le circuit, en comprimant la poche R par les plaques. Une seule plaque F exerçant une pression sur la poche R peut parfois suffire. Le circuit C comprend également au moins une pompe, et plus particulièrement deux pompes, à savoir une pompe de sortie Po et une pompe d'entrée Pi. Il est également envisageable d'utiliser une seule pompe dans le circuit C. Toutefois, l'utilisation de deux pompes permet de garantir un débit constant et une pression constante. Avantageusement un capteur de pression 54 est disposé dans la tête, en communication avec le liquide de couplage et capable de mesurer la pression dans la chambre. Les informations du capteur de pression 54 et des pompes Pi et Po sont envoyées à un régulateur de pression C. Les deux pompes peuvent ainsi être commandées en fonction des informations de pression et optionnellement de débit du fluide. La pression est régulée en appliquant un différentiel de vitesse aux deux pompes Pi, Po. La présence de deux pompes permet aussi de remplir et vider facilement le liquide dans la chambre, en inversant leur rotation. Une purge peut être prévue dans le haut de la poche R pour faciliter l'évacuation du liquide de la chambre et son transfert dans la poche. Ainsi, le liquide injecté dans la tête par le canal d'entrée 41 est réfrigéré et présente une température plus basse que le liquide au niveau du canal de sortie 42. La flèche F2 symbolise le flux thermique injecté par le débit de liquide refroidi. La flèche F3 symbolise le flux thermique extrait par le débit de liquide réchauffé dans la chambre 4. En se plaçant au niveau de la chambre 4, on peut considérer que le canal d'entrée 41 constitue l'entrée de la chambre 4 alors que le canal de sortie 42 constitue la sortie de la chambre 4. Par conséquent, il faudra comprendre dans l'ensemble de la description que le terme « canal d'entrée » signifie « entrée » de la chambre 4 et que le terme « canal de sortie » signifie « sortie » de la chambre 4.

En se référant à la figure unique, on peut voir que la membrane 3 est en contact de la peau S d'un tissu T, avantageusement avec interposition d'un gel de contact G. La membrane 3 est en contact de la peau S au niveau où elle forme la chambre 4. Ainsi, la peau qui a une température de l'ordre de 30°C, transmet de la chaleur au liquide L à l'intérieur de la chambre 4. La flèche F1 symbolise le flux thermique de la peau S vers le liquide L.

D'autre part, la membrane 3 et le boîtier sont également au contact de l'air ambiant qui est en général à une température plus élevée que celle du liquide L. Par conséquent, l'air transmet également de la chaleur au liquide L. La flèche F4 symbolise le flux thermique de l'air ambiant vers le liquide L.

Le transducteur thérapeutique ultrasonore 2, et éventuellement la sonde échographique 6, dissipe également de la chaleur à l'intérieur du liquide L. La flèche F5 symbolise le flux thermique du transducteur 2, et éventuellement de la sonde 6, vers le liquide L. En effet, il faut savoir que tous les transducteurs ultrasonores 2 dissipent une partie de l'énergie fournie en chaleur. L'autre partie est utilisée pour produire des ultrasons. Étant donné que la chaleur dissipée représente de 20 à 50% de l'énergie fournie au transducteur, la quantité de chaleur dissipée à l'intérieur du liquide L est considérable. Sur la figure unique, on a représenté la zone de propagation Z des ultrasons du transducteur 2 dans la chambre 4 par un hachurage. A l'extérieur de la chambre, la zone de propagation Z a été représentée par les traits pointillés. On peut ainsi remarquer que la majeure partie de la chambre 4 est traversée par les ultrasons du transducteur 2. En revanche, on peut également constater que les canaux 41 et 42 et donc leurs capteurs de températures 51 et 52 ne sont pas situés dans la zone de propagation Z des ultrasons du transducteur 2, ni de la sonde échographique 6.

Selon l'invention, un premier capteur de température 51 est disposé à proximité ou au niveau de l'entrée de la chambre 4 et un second capteur de température 52 est disposé à proximité ou au niveau de la sortie de la chambre 4. Plus précisément, le capteur 51 est disposé dans le canal d'entrée 41 et le second capteur 52 est disposé dans le canal de sortie 42. On peut également disposer les capteurs 51 et 52 directement dans la chambre 4 à proximité de l'entrée et de la sortie, mais de préférence hors de la zone de propagation des ondes du transducteur 2, et de la sonde 6. Ainsi, le premier capteur de température 51 permet de mesurer la température Ti du liquide à l'entrée de la chambre 4 alors que le second capteur de température 52 permet de mesurer la température To du liquide à la sortie de la chambre 4. Par exemple, en soustrayant la température To à la température Ti, on obtient un différentiel de température $\Delta$T du liquide de couplage dans la chambre. Ce différentiel de température $\Delta$T va servir à déterminer de manière indirecte la température de la peau.

Pour cela, on utilise l'électronique E à laquelle sont reliés les deux capteurs de température 51 et 52, ainsi que le capteur de débit 53. L'électronique E va recevoir les signaux de température issus des capteurs 51 et 52 et les signaux de débit issus du capteur 53. L'électronique E intègre un logiciel approprié qui permet d'établir le différentiel de température $\Delta$T à partir des températures d'entrée Ti et des températures de sortie To mesurées par les capteurs 51 et 52. L'électronique E va également tenir compte des valeurs de débit, ainsi que de la température ambiante Ta et de la chaleur dissipée par le transducteur 2, et éventuellement la sonde 6 à l'intérieur de la chambre 4. L'électronique E peut ainsi calculer l'énergie calorifique absorbée ou rejetée par le liquide L dans la chambre 4. L'électronique E peut calculer l'ensemble des flux thermique F2 à F5, et à partir de cela, déterminer de manière indirecte le flux thermique F1 et finalement la température de la peau.

Le bilan énergétique à l'aide d'équations physiques est plus parlant. Il faut considérer l'ensemble des échanges thermiques auxquels est soumis le liquide de couplage dans la chambre. Nous pouvons ainsi décomposer ces échanges en quatre flux thermiques principaux, à savoir :

a. F3 - F2 : le différentiel de flux de liquides entrant et sortant. Le liquide froid entrant dans la chambre apporte des calories qui sont plus que compensées par les calories du liquide réchauffé sortant de la chambre.

b. Le flux thermique F1 au travers de la membrane : la température de la peau étant plus élevée que celle du liquide L, il y a un flux thermique (transfert de calories) de la peau vers le liquide de la chambre.

c. Le flux thermique de l'air ambiant F4. La température de l'air ambiant étant supérieure à la température du liquide, des calories de l'air ambiant sont transférées au liquide dans la chambre.

d. Le flux électro-acoustique F5 : le rendement du transducteur n'étant pas parfait, le transducteur chauffe et transmet des calories au liquide dans la chambre.

a. F3 - F2 : ceci correspond à la puissance évacuée par le liquide. F3 représente la puissance sortant alors que F2 représente la puissance entrante. Au total, on peut calculer la puissance évacuée :

*Puissance évacuée Pe = 4, 1855. (To.Do-Ti.Di).*

Ti : Température de l'eau entrante en °C.
Di : Débit massique de l'eau entrante en g/s.
To : Température de l'eau réchauffée sortante en °C.
Do : Débit massique de l'eau sortante en g/s. en régime fluidique stationnaire
Do = Di

b. F1 : la température de la peau étant plus élevée que celle du liquide, le flux thermique de la peau vers le liquide peut s'exprimer de la manière suivante :

$$Puissance\_fournie = \frac{\lambda_m \cdot S}{E_m} \cdot (T_s - T_l)$$

Ts = Température moyenne de la peau en °C sur la surface d'échange S.
Tl = Température moyenne du liquide dans la chambre en °C.
S = Surface d'échange de la membrane avec la peau en cm$^2$.
Em = Epaisseur membrane en cm.
λm = Conductivité thermique de la membrane en W/°C/cm.
Il est possible de rassembler toutes les constantes en une seule constante $k_m$ en W/°C :

$$Puissance\_fournie = k_m \cdot (T_s - T_l) \quad \text{avec} \quad k_m = \frac{\lambda_m \cdot S}{E_m}$$

c. F4 : *Puissance fournie = Ka.(Ta - Tl)*
Ta = Température moyenne de l'air ambiant en °C proche de la tête de traitement.
Tl = Température moyenne du liquide dans la chambre en °C.
Ka = Constante lié à la géométrie et aux matériaux utilisés pour la tête de traitement en W/°C.

F5: $Puissance\_fournie = K_e \cdot P_e \cdot \left( \dfrac{tOn}{tOn + tOff} \right)$

tOn : Durée d'émission des pulses HIFU en s.
tOff : Durée de repos entre les pulses HIFU en s.
Pe : Puissance électrique en W envoyé au transducteur durant les pulses.
Ke : Coefficient correctif tenant compte du rendement acoustique du transducteur sans unité et d'effet de réverbération acoustique.

A partir de ces puissances, on peut établir l'équation du bilan thermique en régime stable. Celle-ci s'exprime comme suit :

$$K_e \cdot P_e \cdot \left( \frac{tOn}{tOn + tOff} \right) + Ka \cdot (T_a - T_l) + k_m \cdot (T_s - T_l) = 4.1855 \cdot (T_o \cdot D_o - T_i \cdot D_i)$$

On peut ainsi en déduire la température de la peau comme suit :

$$T_s = T_l + \frac{4.1855 \cdot (T_o \cdot D_o - T_i \cdot D_i) - K_e \cdot P_e \cdot \left(\dfrac{tOn}{tOn + tOff}\right) - Ka \cdot (T_a - T_i)}{k_m}$$

On peut également établir l'équation du bilan thermique en régime transitoire. Si l'équation précédente du bilan thermique n'est pas équilibrée, la température du liquide dans la tête varie. Elle augmente si la puissance fournie est supérieure à la puissance évacuée et inversement.

$$PuissanceFournie - PuissanceEvacuée = 4.1855 \cdot \frac{dT_l}{dt} \cdot M_l$$

$dT_l/dt$ : Dérivée par le temps de la température du liquide dans la chambre en °C/s.
$M_l$ : Masse du liquide dans la chambre en g.

$$K_e \cdot P_e \cdot \left(\frac{tOn}{tOn + tOff}\right) + Ka \cdot (T_a - T_i) + k_m \cdot (T_s - T_l) - 4.1855 \cdot (T_o \cdot D_o - T_i \cdot D_i) = 4.1855 \cdot \frac{dT_l}{dt} \cdot M_l$$

Nous pouvons donc en déduire la température de la peau par :

$$Ts = T_l + \frac{4.1855 \cdot \dfrac{dT_l}{dt} \cdot M_l + 4.1855 \cdot (T_o \cdot D_o - T_i \cdot D_i) - K_e \cdot P_e \cdot \left(\dfrac{tOn}{tOn + tOff}\right) - Ka \cdot (T_a - T_i)}{k_m}$$

La plupart des données sont des constantes qui peuvent être déterminées par paramétrage. Quant aux températures Ti et To, elles sont mesurées par les capteurs de température 51 et 52. Le débit est mesuré par le capteur 53 ou connu à l'avance. La température ambiante peut être mesurée par un thermomètre. La température moyenne Tm peut être prise comme la moyenne de Ti et To. Le flux F4 en fonction de Tm et Ti ou To peut être obtenu par étalonnage.

Le logiciel embarqué dans l'électronique E va effectuer les calculs d'équations de bilan thermique ci-dessus et déterminer la température de la peau du tissu à traiter. De manière très générale, l'électronique E calcule le différentiel de température ΔT et le corrèle avec le débit du liquide, la température ambiante extérieure, la chaleur dissipée par le transducteur dans le liquide pour déterminer de manière indirecte la température de la peau. Grâce à ce différentiel de température mesuré et l'électronique E associée, il est possible de savoir si le contact entre la membrane 3 et la peau S est optimal, ou au contraire si la membrane est décollée localement ou entièrement de la peau. On peut par exemple déplacer la membrane sur la peau. Si la surface de contact est grande, et donc le contact de bonne qualité, le déplacement de la membrane ne se traduira que par une variation minimale du différentiel de température. Au contraire, si la surface est petite, et donc le contact de mauvaise qualité, le déplacement de la membrane va modifier la zone de contact, et engendrer une variation considérable du différentiel de température. On peut également se servir du différentiel de température pour déterminer si le transducteur fonctionne réellement. En effet, si le transducteur est en panne soit il ne reçoit pas d'énergie électrique, il n'y a pas de chaleur dissipée dans le liquide et le différentiel de température ne va que faiblement varier, soit il reçoit de l'énergie électrique mais n'émet pas d'ultrasons, et dans ce cas, le différentiel de température va fortement augmenter. Et lorsque le transducteur est activé et émet des ultrasons, l'estimation de la température de surface d'application va permettre d'alerter l'opérateur d'un échauffement excessif au niveau de la peau. A cet effet, il est avantageux, lors d'une phase initiale dans laquelle le transducteur n'est pas activé, d'appliquer la membrane sur la peau du tissu à traiter et de faire circuler le liquide dans la chambre jusqu'à ce que la température de la peau se stabilise de sorte que la chaleur échangée entre la peau et le liquide de la chambre soit proche du minimal. Dès que l'on a atteint une température de peau stable, le traitement peut commencer en toute sécurité cutanée. De même il est possible de détecter les mouvements du patient en surveillant la pression dans la chambre. Par exemple, si la tête s'éloigne de la peau, la pression va chuter et le ballonnet va se remplir pour compenser cette pression. En détectant les variations de volume dans le ballonnet (via les variations de vitesse des pompes) on voit si le couplage est modifié.

Étant donné que les différentiels de températures mesurés sont généralement faibles, il est important que les mesures soient précises. Pour annuler les différences, on aura intérêt, par exemple lors d'une phase de calibration, d'inverser le sens du flux du liquide et d'effectuer ainsi deux mesures, l'une en sens direct et l'autre en sens inverse. D'autre part, afin de réduire au minimum les échanges thermiques, il est avantageux de réaliser le boîtier avec un matériau connu pour ses qualités d'isolation thermique comme par exemple le PEEK®.

En se référant à nouveau à la figure unique, on peut voir que l'appareil de traitement thérapeutique peut comprendre un commutateur K qui permet d'activer et de désactiver le transducteur thérapeutique 2. Le commutateur K est ici couplé à l'électronique E. Selon une caractéristique intéressante de l'invention, le différentiel de température $\Delta T$, ou la température Ts de la peau indirectement mesurée à partir des différentiels de température $\Delta T$, va servir de valeur de déclenchement du commutateur K. Plus précisément, le commutateur K va désactiver le transducteur 2 dès lors que $\Delta T$ ou Ts va atteindre une valeur plafond prédéterminée indicative d'une surchauffe de la peau. D'autre part, le commutateur K va activer le transducteur 2 dès lors que $\Delta T$ ou Ts est redescendu à une valeur seuil prédéterminée indicative d'une température normale ou basse de la peau. En d'autres termes, les séquences d'activation et de repos du transducteur sont imposées par le commutateur K qui est commandé par l'électronique E qui se sert du différentiel de température ou de la température de la peau pour déclencher le commutateur K. L'opérateur chargé du traitement thérapeutique n'a alors même plus besoin de se soucier de la température de la peau du tissu à traiter, étant donné que le transducteur 2 va automatiquement être activé et désactivé en fonction de la température de la peau ou du différentiel de température. Les risques d'endommagement de la peau sont ainsi réduits.

L'estimation d'une valeur de température de surface appliquée anormalement basse (inférieure à la température du liquide par exemple) correspond à une défaillance du transducteur, plus précisément à son absence de dissipation calorique alors que celle-ci est attendue.

Le commutateur K est ici automatiquement et directement déclenché par l'électronique E. En variante, il est également possible de découpler le commutateur K de l'électronique E. Dans ce cas, c'est à l'opérateur chargé du traitement thérapeutique d'activer et de désactiver le transducteur dès lors que la température de la peau ou le différentiel de température atteint la valeur plafond et la valeur seuil.

En variante, il est possible de diminuer momentanément le débit du circuit de fluide pour augmenter le $\Delta T$ et améliorer la précision de la mesure.

Grâce à l'invention, on peut se servir d'un différentiel de température mesuré à l'aide de deux capteurs pour déterminer de manière indirecte la température de la surface d'application d'un tissu à traiter.

**Revendications**

1. Tête de traitement thérapeutique pour traiter des tissus vivants (T), la tête comprenant un boîtier (1), un transducteur thérapeutique ultrasonore (2) monté sur le boîtier et une membrane (3) montée sur le boîtier (1), la membrane (3) étant destinée à venir en contact avec une surface d'application (S) du tissu (T), telle que la peau, la membrane (3), le transducteur (2) et le boîtier (1) définissant ensemble une chambre (4) remplie d'un liquide de couplage acoustique (L) qui circule à travers la chambre entre une entrée (41) et une sortie (42), le transducteur (2) étant disposé de manière à émettre des ultrasons à travers une zone de propagation (Z) de cette chambre en direction de la membrane (3),
**caractérisée en ce qu'**un premier capteur de température (51) est disposé à proximité de l'entrée (41) et un second capteur de température (52) est disposé à proximité de la sortie (42), les capteurs (51, 52) délivrant des signaux représentatifs des températures.

2. Tête de traitement thérapeutique selon la revendication 1, dans laquelle les capteurs de température (51, 52) sont situés hors de la zone de propagation (Z).

3. Appareil de traitement thérapeutique comprenant une tête de traitement selon la revendication 1 ou 2, l'appareil comprenant une électronique (E) recevant les signaux de température délivrés par les capteurs de température (51, 52) pour établir un différentiel de température ($\Delta T$) servant à déterminer la température (Ts) de la surface d'application (S).

4. Appareil de traitement thérapeutique selon la revendication 3, dans lequel l'électronique (E) calcule l'énergie calorifique absorbée ou rejetée par le liquide (L) dans la chambre (4).

5. Appareil de traitement thérapeutique selon la revendication 3 ou 4, comprenant un capteur de débit de liquide (53) délivrant des signaux de débit à l'électronique (E) pour calculer l'énergie calorique absorbée ou rejetée par le liquide (L) dans la chambre (4).

**6.** Appareil de traitement thérapeutique selon la revendication 3, 4 ou 5, comprenant des moyens de refroidissement (F) pour refroidir le liquide (L) avant de l'injecter dans la chambre (4) à travers l'entrée (41).

**7.** Appareil de traitement selon la revendication 6, dans lequel les moyens de refroidissement comprennent au moins une plaque de refroidissement (F) exerçant une pression réglable sur une poche réservoir (R) de liquide (L).

**8.** Appareil de traitement selon l'une quelconque des revendications précédentes, comprenant au moins une pompe (Pi, Po) et un capteur de pression (54) couplés à un régulateur de pression (C) pour réguler la pression dans la chambre (4).

**9.** Appareil de traitement thérapeutique selon l'une quelconque des revendications 3 à 7, comprenant des moyens de commutation (K) du transducteur (2) couplés à l'électronique (E), les moyens de commutation désactivant le transducteur lorsque le différentiel de température ($\Delta$T) atteint une valeur plafond prédéterminée indicative d'une surchauffe de la surface d'application (S).

**10.** Appareil de traitement thérapeutique selon la revendication 9, dans lequel les moyens de commutation (K) activent le transducteur (2) lorsque le différentiel de température ($\Delta$T) est redescendu à une valeur seuil prédéterminée indicative d'une température normale de la surface d'application (S).

**Claims**

**1.** A therapeutic treatment head for treating living tissue (T), the head comprising a casing (1), an ultrasound therapeutic transducer (2) mounted on the casing, and a diaphragm (3) mounted on the casing (1), the diaphragm (3) being designed to come into contact with an application surface (S) of the tissue (T), such as skin; the diaphragm (3), the transducer (2), and the casing (1) together defining a chamber (4) that is filled with an acoustic coupling liquid (L) that flows through the chamber between an inlet (41) and an outlet (42); the transducer (2) being placed in such a manner as to emit ultrasound through a propagation zone (Z) of the chamber towards the diaphragm (3); the head being **characterized in that** a first temperature sensor (51) is placed close to the inlet (41) and a second temperature sensor (52) is placed close to the outlet (42), the sensors (51, 52) delivering signals that are representative of temperatures.

**2.** A therapeutic treatment head according to claim 1, wherein the temperature sensors (51, 52) are situated outside the propagation zone (Z).

**3.** A therapeutic treatment appliance including a treatment head according to claim 1 or claim 2, the appliance including electronics (E) receiving the temperature signals delivered by the temperature sensors (51, 52) to establish a temperature difference ($\Delta$T) that is used for determining the temperature ($T_s$) of the application surface (S).

**4.** A therapeutic treatment appliance according to claim 3, wherein the electronics (E) calculates the heat energy absorbed or rejected by the liquid (L) in the chamber (4).

**5.** A therapeutic treatment appliance according to claim 3 or claim 4, including a liquid flow rate sensor (53) delivering flow rate signals to the electronics (E) in order to calculate the heat energy absorbed or rejected by the liquid (L) in the chamber (4).

**6.** A therapeutic treatment appliance according to claim 3, 4, or 5, including cooler means (F) for cooling the liquid (L) prior to injecting it into the chamber (4) through the inlet (41).

**7.** A treatment appliance according to claim 6, wherein the cooler means comprise at least one cooler plate (F) exerting adjustable pressure on a reservoir pouch (R) of liquid (L).

**8.** A treatment appliance according to any preceding claim, including at least one pump ($P_i$, $P_o$) and a pressure sensor (54) coupled to a pressure regulator (C) for regulating the pressure in the chamber (4).

**9.** A therapeutic treatment appliance according to any one of claims 3 to 7, including switch means (K) coupled to the electronics (E), the switch means deactivating the transducer (2) when the temperature difference ($\Delta$T) reaches a predetermined ceiling value indicative of the application surface (S) overheating.

**10.** A therapeutic treatment appliance according to claim 9, wherein the switch means (K) activate the transducer (2) when the temperature difference ($\Delta$T) has come back below a predetermined threshold value indicative of a normal temperature for the application surface (S).

**Patentansprüche**

**1.** Therapeutischer Behandlungskopf zur Behandlung von Lebendgewebe (T), wobei der Kopf ein Gehäuse (1), einen auf dem Gehäuse angebrachten therapeutischen Ultraschallwandler (2) und eine auf dem Gehäuse (1) angebrachte Membran (3) umfasst, wobei die Membran (3) dazu bestimmt ist, mit einer Anwendungsfläche (S) des Gewebes (T), wie der Haut, in Kontakt zu kommen, wobei die Membran (3), der Wandler (2) und das Gehäuse (1) zusammen eine Kammer (4) definieren, die mit einer akustischen Kopplungsflüssigkeit (L) gefüllt ist, die zwischen einem Eingang (41) und einem Ausgang (42) durch die Kammer fließt, wobei der Wandler (2) so angeordnet ist, dass er durch einen Übertragungsbereich (Z) dieser Kammer Ultraschallwellen in Richtung zur Membran (3) ausstrahlt, **dadurch gekennzeichnet, dass** ein erster Temperatursensor (51) in der Nähe des Eingangs (41) und ein zweiter Temperatursensor (52) in der Nähe des Ausgangs (42) angeordnet ist, wobei die Sensoren (51, 52) Signale ausgeben, die Temperaturen darstellen.

**2.** Therapeutischer Behandlungskopf nach Anspruch 1, wobei sich die Temperatursensoren (51, 52) außerhalb des Übertragungsbereichs (Z) befinden.

**3.** Therapeutisches Behandlungsgerät, aufweisend einen Behandlungskopf nach Anspruch 1 oder 2, wobei das Gerät eine Elektronik (E) umfasst, die die von den Temperatursensoren (51, 52) zugeführten Temperatursignale empfängt, um eine Temperaturdifferenz ($\Delta$T) zu erstellen, die dazu dient, die Temperatur (Ts) der Anwendungsoberfläche (S) zu ermitteln.

**4.** Therapeutisches Behandlungsgerät nach Anspruch 3, wobei die Elektronik (E) die von der Flüssigkeit (L) in der Kammer (4) aufgenommene oder abgegebene Wärmeenergie berechnet.

**5.** Therapeutisches Behandlungsgerät nach Anspruch 3 oder 4, aufweisend einen Flüssigkeit-Durchflusssensor (53), der der Elektronik (E) Durchflusssignale zuführt, um die von der Flüssigkeit (L) in der Kammer (4) aufgenommene oder abgegebene Wärmeenergie zu berechnen.

**6.** Therapeutisches Behandlungsgerät nach Anspruch 3, 4 oder 5, das eine Kühleinrichtung (F) zum Kühlen der Flüssigkeit (L), bevor sie über den Eingang (41) in die Kammer (4) eingespritzt wird, aufweist.

**7.** Behandlungsgerät nach Anspruch 6, wobei die Kühleinrichtung mindestens eine Kühlplatte (F) aufweist, die einen einstellbaren Druck auf eine Speichertasche (R) für die Flüssigkeit (L) ausübt.

**8.** Behandlungsgerät nach einem der vorhergehenden Ansprüche, das mindestens eine Pumpe (Pi, Po) und einen Drucksensor (54) aufweist, die an einen Druckregler (C) gekoppelt sind, um den Druck in der Kammer (4) zu regeln.

**9.** Therapeutisches Behandlungsgerät nach einem der Ansprüche 3 bis 7, aufweisend eine Umschalteinrichtung (K) für den Wandler (2), die an die Elektronik (E) angeschlossen ist, wobei die Umschalteinrichtung den Wandler deaktiviert, wenn die Temperaturdifferenz ($\Delta$T) einen vorbestimmten Grenzwert erreicht, der eine Überhitzung der Anwendungsoberfläche (S) anzeigt.

**10.** Therapeutisches Behandlungsgerät nach Anspruch 9, wobei die Umschalteinrichtung (K) den Wandler (2) aktiviert, wenn die Temperaturdifferenz ($\Delta$T) wieder auf einen vorbestimmten Schwellenwert gesunken ist, der eine normale Temperatur der Anwendungsoberfläche (S) anzeigt.

Fig. unique

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0205897 A